# EUROPÄISCHE PATENTSCHRIFT

(11) **EP 3 340 896 B1**
(45) Veröffentlichungstag und Bekanntmachung des Hinweises auf die Patenterteilung: **03.07.2019**
(21) Anmeldenummer: 16757012.6
(22) Anmeldetag: 24.08.2016
(51) Int. Cl.: A61B 17/22, A61F 7/12

(54) **MEDIZINISCHES ASPIRATIONSSYSTEM UND MEDIZINISCHES THROMBEKTOMIESYSTEM**
MEDICAL ASPIRATION SYSTEM AND MEDICAL THROMBECTOMY SYSTEM
SYSTÈME MÉDICAL D'ASPIRATION ET SYSTÈME MÉDICAL DE THROMBECTOMIE

(30) Priorität: 25.08.2015 DE 102015114044
(43) Veröffentlichungstag der Anmeldung: 04.07.2018
(73) Patentinhaber: Adceris GmbH & Co. KG, 75177 Pforzheim (DE)
(72) Erfinder: CATTANEO, Giorgio, 76199 Karlsruhe (DE)
(74) Vertreter: Meissner Bolte Partnerschaft mbB
(86) Internationale Anmeldenummer: PCT/EP2016/069965
(87) Internationale Veröffentlichungsnummer: WO 2017/032792

(56) Entgegenhaltungen:
- EP-A1- 2 842 604
- DE-A1-102013 104 021
- US-A- 5 554 136
- US-A1- 2005 004 503
- US-A1- 2008 319 376
- US-A1- 2014 228 869
- US-A1- 2015 018 904
- US-A1- 2015 173 782

## Beschreibung

Die Erfindung bezieht sich auf ein medizinisches Aspirationssystem mit einem Katheter mit wenigstens zwei Lumen, wobei ein erstes Lumen ein Aspirationslumen bildet, das mit einer Aspirationseinheit zum Absaugen von Stoffen aus einem Blutgefäß durch eine Eintrittsöffnung des Aspirationslumens verbunden ist. Ein zweites Lumen bildet ein Infusionslumen, durch das ein Stoff dem Blutgefäß durch eine Austrittsöffnung des Infusionslumens zuführbar ist. Die Austrittsöffnung des Infusionslumens ist in einem Bereich zwischen der Eintrittsöffnung des Aspirationslumens und einer Position proximal zur Eintrittsöffnung des Aspirationslumens angeordnet.

Ein solches Aspirationssystem ist bspw. aus US 2005/0004503 A1 bekannt. Das in US 2005/0004503 A1 beschriebene Aspirationssystem umfasst einen Katheter mit wenigstens zwei Lumen. Das erste Lumen bildet ein Aspirationslumen und hat die Funktion, durch eine Eintrittsöffnung Stoffe aus einem Blutgefäß abzusaugen. Das zweite Lumen bildet ein Infusionslumen, das mit einer Kühleinheit verbunden ist und eine Austrittsöffnung aufweist, durch die dem Blutgefäß eine gekühlte Flüssigkeit zuführbar ist.

Ein weiteres System, das ein Aspirations- und ein Infusionslumen umfasst, ist aus US 2014/0228869 A1 bekannt.

Ein weiteres Aspirationssystem ist beispielsweise aus US 5,554,136 bekannt.

Das aus US 5,554,136 bekannte Aspirationssystem wird dazu verwendet, um Medikamente zu verabreichen und Thromben durch Aspiration zu entfernen. Dazu weist das Aspirationssystem einen Katheter mit zwei Arbeitslumen auf, die distal geschlossen sind und für die Aspiration und Infusion Schlitzventile aufweisen. Das Problem bei derartigen Kathetern besteht darin, dass diese wegen der Schlitzventile konstruktiv aufwändig sind und nur zur Aspiration in Verbindung mit der Verabreichung von Medikamenten ausgelegt sind.

US 5,554,136 schlägt vor, den Katheter im Bereich der Schlitzventile mit einem speziellen Profil und einer bestimmten Steifigkeit zu versehen. Dies ändert aber nichts daran, dass derartige Aspirationssysteme kompliziert sind.

Weitere Systeme sind aus US 2008/0319376 A1 und EP 2 842 604 A1 bekannt.

Der Erfindung liegt die Aufgabe zu Grunde, ein Aspirationssystem der eingangs genannten Art mit Blick auf die Komplexität und schonende Behandlung zu verbessern.

Erfindungsgemäß wird diese Aufgabe durch ein Aspirationssystem mit den Merkmalen des Anspruchs 1 gelöst.

Die Erfindung beruht auf dem Gedanken, ein medizinisches Aspirationssystem anzugeben, das einen Katheter mit wenigstens zwei Lumen aufweist, wobei ein erstes Lumen ein Aspirationslumen bildet, das mit einer Aspirationseinheit zum Absaugen von Stoffen aus einem Blutgefäß verbunden ist. Das Absaugen der Stoffe erfolgt durch eine Eintrittsöffnung des Aspirationslumens. Ein zweites Lumen bildet ein Infusionslumen, durch das ein Stoff dem Blutgefäß zuführbar ist. Die Zufuhr erfolgt durch eine Austrittsöffnung des Infusionslumens. Die Austrittsöffnung des Infusionslumens ist in einem Bereich zwischen einer der Eintrittsöffnung des Aspirationslumens und einer Position proximal zur Eintrittsöffnung des Aspirationslumens angeordnet. Das Infusionslumen ist mit einer Kühleinheit verbunden, insbesondere fluidverbunden, die eine gekühlte Flüssigkeit zur Verabreichung durch das Infusionslumen bereitstellt.

Die Eintrittsöffnung des Aspirationslumens und die Austrittsöffnung des Infusionslumens befinden sich jeweils im distalen Katheterbereich, d.h. im Gebrauch auf der Blutseite. Der Begriff Eintrittsöffnung bezieht sich auf die Aspirationsfunktion und den Eintritt der angesaugten Stoffe in das Aspirationslumen. Der Begriff Austrittsöffnung bezieht sich auf die Infusionsfunktion und den Austritt der zugeführten Flüssigkeit aus dem Infusionslumen.

Die Erfindung hat den Vorteil, dass die Verbindung des Infusionslumens mit einer Kühleinheit durch die protektive Wirkung der Hypothermiebehandlung eine besonders schonende Entfernung von Thromben aus dem Blutkreislauf ermöglicht. Dazu stellt die Kühleinheit eine Flüssigkeit mit einer Temperatur bereit, die niedriger als die Körpertemperatur ist. Diese Flüssigkeit wird durch das mit der Kühleinheit verbundene Infusionslumen verabreicht. Damit wird auf einfache und wirksame Weise eine Kühlung der behandelten Blutgefäße erreicht. Die Kühlung kann zeitgleich mit der Aspiration stattfinden, so dass mögliche durch die Aspiration hervorgerufene Komplikationen verringert werden. Die Erfindung hat den Vorteil, dass eine wirksame Kühlung ohne zusätzliche Kühlelemente erreicht wird. Die gekühlte Flüssigkeit wird erfindungsgemäß durch das Infusionslumen direkt in das Blutgefäß abgegeben. Dazu ist vorteilhafterweise das Infusionslumen distal offen. Die Kühleinheit ist mit dem Infusionslumen fest oder lösbar verbunden. In jedem Fall bildet die Kühleinheit Teil des erfindungsgemäßen Aspirationssystems.

Der komplizierte Aufbau des Katheters aus dem Stand der Technik entfällt. Die Erfindung ermöglicht vielmehr den Einsatz eines mehrlumigen Katheters, d.h. eines Katheters mit wenigstens zwei Lumen, wobei zumindest das Aspirationslumen, insbesondere das Aspirationslumen und das Infusionslumen, am distalen Katheterende offen ist/sind. Der Thrombus kann dadurch in das Aspirationslumen eingesaugt oder zusammen mit einer Rekanalisationseinheit und/oder Thrombektomie-Einheit in das Aspirationslumen eingebracht werden. Der Thrombus befindet sich dann im Aspirationslumen und kann entfernt werden.

Im Gegensatz zu einer Anordnung, bei der das Infusionslumen distal zum Aspirationslumen endet, hat die erfindungsgemäße Anordnung der Austritts- und Eintrittsöffnung im Zusammenhang mit der Kühleinheit den Vorteil, dass die gekühlte Flüssigkeit, die aus dem Infusionslumen austritt nicht oder zumindest in verringertem Maße durch das Aspirationslumen abgesaugt wird. Dadurch wird eine wirksame Kühlung erreicht.

Das ist besonders dann der Fall, wenn Gefäßabzweigungen oder Kollateralgefäße sich auf der Höhe des Infusionslumensaustritts oder zwischen dem Infusionslumenaustritts und dem Aspirationslumeneintritts befinden. Es wird somit möglich, während der Aspiration die Kollateralgefäße mit kalter Infusion zu versorgen. Diese Anordnung ist in allen Phasen der Prozedur wichtig, selbst wenn keine Aspiration stattfindet. Das Aspirationslumen kann zB für die Zufuhr von distalen Kathetern, zB Aspirationskathetern und Intermediate Kathetern und Systemen für die mechanische Thrombektomie, benutzt werden. Während der Zufuhr wird das Aspirationslumen mit Infusion mit geringer Flussrate gespült, damit die Zufuhr reibungslos erfolgt. In dieser Phase ist es vorteilhaft, wenn die kalte Infusion durch ein getrenntes Lumen erfolgt, das nicht durch einen solchen Katheter oder andere Thrombektomie-Systeme teilweise gesperrt ist. Auf dieser Weise sind höhere Flussraten an kalter Infusion möglich, als wenn die kalte Infusion im gleichen Lumen erfolgen würde.

Das Infusionslumen unterscheidet sich vom Aspirationslumen im Durchmesser. Das Aspirationslumen hat einen größeren Durchmesser als das Infusionslumen. Außerdem weist das Aspirationslumen vorzugsweise eine Katheterwand auf, die dimensionsstabiler als die Katheterwand des Infusionslumen ist. Dadurch wird verhindert, dass der Katheter beim Aspirieren kollabiert. Da beim Zuführen von Flüssigkeiten Überdruck im Infusionslumen herrscht, stellt sich das Problem der Kollabierung des Infusionslumens nicht oder nur in geringerem Maße.

Insbesondere die Katheterwand des Aspirationslumens kann verstärkt werden, zB durch ein Metall und/oder ein anderes, zusätzliches Kunststoff-Material. Eine reibungsvermindernde Innenschicht kann bereitgestellt werden. Somit ist die Zufuhr von weiteren Katheter, Aspirationskathetern und Intermediate Kathetern oder Systemen für die Thrombektomie begünstigt. Auch das Infusionslumen kann verstärkt werden, durch ein Metall und/oder durch ein weiteres Kunststoffmaterial. Die Variante ohne Metall, also nur mit einem zusätzlichen Material, ist bei dem Infusionslumen zu bevorzugen, weil das Lumen wegen der kleinen Dimensionen nicht zum Kollabieren neigt. Beide Lumen können durch ein weiteres Material verstärkt werden. Alternativ ist nur ein Lumen verstärkt und das andere Lumen ist nicht verstärkt. Im optimalen Fall weist das Aspirationslumen wenigstens eine Metallverstärkung und eine reibungsvermindernde Kunststoffschicht auf. Das Infusionslumen weist ein zusätzliches Verstärkungsmaterial auf. Bevorzugt ist das Infusionslumen in demselben Kunststoffmaterial eingebettet, in das auch das Aspirationslumen einbettet ist.

Dabei kann es in direktem Kontakt mit dem Kunststoff stehen (d.h. kein weiteres Material zur Verstärkung ist vorhanden) oder es weist ein Verstärkungsmaterial auf. Es kann auch sein, dass die gleiche Metallverstärkung beide Lumen gleichzeitig umschließt. In diesem Fall ist ein weiteres Kunststoffmaterial vorgesehen, das die Metallverstärkung umschließt. Der Innenkunststoff ist zweilumig, zB aus PTFE, oder aus einem anderen Material. Das Aspirationslumen kann eine Innenschicht aus Teflon aufweisen.

Für alle Ausführungsformen der Erfindung gilt, dass in das Aspirationslumen bzw. Instrumentenlumen grundsätzlich ein weiterer (Mikro-)Katheter geschoben werden kann, der distal aspiriert oder mit Hilfe selbstsexpandierbarer Vorrichtungen zur mechanischen Thrombektomie den Thrombus aufnimmt. Dieser (Mikro-)Katheter wird dann in das Aspirationslumen bzw. Instrumentenlumen zurückgezogen, während im Aspirationslumen bzw. Instrumentenlumen gesaugt wird oder auch nicht. Durch das Aspirationslumen bzw. Instrumentenlumen kann ein Thrombus entfernt oder zurückgezogen werden, unabhängig davon, ob das Zurückziehen durch einen Unterdruck, zB durch eine Spritze oder durch eine andere Aspirationsvorrichtung, oder rein mechanisch, unter anderem durch die mechanische Thrombektomie, bewirkt wird. Es ist auch möglich, dass mehrere (Mikro-)Katheter in "teleskopischem" Aufbau angeordnet sind. Nur einer der (Mikro-)Katheter oder alle (Mikro-)Katheter können ein Infusionslumen zur Verabreichung von kalter Flüssigkeit aufweisen.

Bevorzugte Ausführungen der Erfindung Gegenstand der Unteransprüche.

Vorzugsweise sind das Aspirationslumen und das Infusionslumen durch eine ventilfreie Wandung fluiddicht getrennt. Der Katheter kann als einfacher durchgängiger Schlauch mit einem Mehrfachlumen ausgebildet sein. Zwischen den einzelnen Mehrfachlumen bestehen keine Verbindungen, was die Herstellung des Katheters vereinfacht. Durch die fluiddichte Trennung der Lumen wird außerdem vermieden, dass bei der Verabreichung der Infusionsflüssigkeit im Aspirationslumen befindliche Thrombenteile aus dem Katheter geschwemmt werden.

Insbesondere kann die Infusion unbeeinflusst von der Thrombenentfernung fortgesetzt werden, auch nach Abschluss der Thrombusentfernung. Weitere Kontrollen des Gefäßzustandes sind zB durch Verabreichung von Kontrastmittel durch das Aspirationslumen möglich, während die Infusion durch das Infusionslumen erfolgt.

Die Herstellung des Katheters wird vereinfacht, wenn das Aspirationslumen und das Infusionslumen einteilig ausgebildet sind.

Alternativ können die wenigstens zwei Lumen des Katheters ineinander längsverschieblich angeordnet sein. Die Lumen können insbesondere koaxial ineinander und zueinander relativ längsverschieblich positioniert sein. Konkret kann vorgesehen sein, dass das Aspirations- oder Instrumentenlumen bzw. das Funktionslumen koaxial innerhalb des Infusionslumens anordnet ist.

Es wird betont, dass unter dem Begriff Katheter auch "Schleusen" gemeint sind. Schleusen werden mittels Mandrels, auch "Dilators" oder "Dilatatoren" genannt, in dem Gefäß direkt platziert, ohne Verwendung von weiteren Außenschläuchen. Die Verwendung als Schleuse ist besonders vorteilhaft, weil die Dimensionen einer Schleuse größer gewählt werden können als bei einem Katheter. Somit ist der Einsatz eines größeren Lumens sowohl für die Aspiration als auch für die Infusion denkbar.

Bei einer Ausführungsform beträgt der Bereich zwischen der Eintrittsöffnung des Aspirationslumens und der Position proximal zur Eintrittsöffnung des Aspirationslumens wenigstens 0 cm, insbesondere wenigstens 5 cm, insbesondere wenigstens 10 cm, insbesondere wenigstens 20 cm. Je größer der Abstand in der Austrittsöffnung des Infusionslumens von der Eintrittsöffnung des Aspirationslumens, umso weniger gekühlte Flüssigkeit wird in das Aspirationslumen eingesaugt und kann in Gefäßabzweigungen fließen, ohne aspiriert zu werden. Der Abstand der Austrittsöffnung des Infusionslumens von der Eintrittsöffnung des Aspirationslumens sollte nicht mehr als 30 cm betragen, damit die Wirksamkeit der Kühlung im Bereich der Eintrittsöffnung des Aspirationslumens beibehalten wird.

Die Länge des Katheters kann mindestens 80 cm, insbesondere mindestens 90 cm betragen (Position in der ACC). Der Katheter kann alternativ eine Länge von mindestens 90 cm, mindestens 100 cm, mindestens 110 cm aufweisen (Position in der ACI, MCA). Der Durchmesser des Katheters kann mindestens 7 Fr, insbesondere mindestens 8 Fr und höchstens 11 Fr, insbesondere höchstens 10 Fr, insbesondere höchstens 9 Fr betragen (Position in der ACC). Alternativ kann der Katheter einen Durchmesser zwischen 5 und 7 Fr, vorzugsweise von 6 Fr aufweisen (Position in der ACI, MCA).

Wenn der Katheter als "Schleuse" betrachtet wird, kann das Aspirationslumen mit einem Katheter mit Durchmesser von mindestens 4 Fr, 5 Fr oder 6 Fr kompatibel sein. 6Fr ist dabei die Obergrenze. Als Schleuse sollte der Außendurchmesser im optimalen Fall maximal 13 Fr, insbesondere maximal 12 Fr, insbesondere maximal 11 Fr, sein.

Die Querschnittsfläche des Infusionslumens kann zwischen 0,16 mm² und 0,4 mm² betragen. Damit kann ein für die Kühlung ausreichend großer Volumenstrom durch das Infusionslumen transportiert werden.

Für Anwendungen, in denen die kalte Flüssigkeit in sehr kurzer Zeit verabreicht wird, kann das Lumen einen Durchmesser zwischen 0,16 mm² und 1 mm² aufweisen, insbesondere zwischen 0,2 mm² und 0,8 mm², insbesondere zwischen 0,3 mm² und 0,6 mm².

Wenn die Kühleinheit und das Infusionslumen lösbar verbunden sind, kann die Kühleinheit zumindest temporär durch eine andere Funktionseinheit ersetzt werden, wobei die Kühleinheit Teil des Aspirationssystems bleibt. Diese Ausführungsform hat unterstützende Wirkung, um die Entfernung des Thrombus zu erleichtern. Beispielsweise kann durch die Zufuhr von Lyse der Thrombus verkleinert, zerteilt oder weicher gemacht werden. Die Funktionseinheit kann eine mit Lyse oder mit Kontrastmittel gefüllte Zufuhreinheit umfassen, die mit dem Infusionslumen verbunden wird. Auch hier gilt, dass eine zeitgleiche Behandlung eines Patienten während der Aspiration möglich ist. Durch die Lyse wird die Entfernung von Thromben mittels Aspiration unterstützt. Der Einsatz von Kontrastmittel erleichtert die Positionierung des Aspirationslumen bezogen auf den zu entfernenden Thrombus. Nach der Aspiration kann eine angiografische Kontrolle durchgeführt werden, um die Offenheit des Gefäßes prüfen und ggf. weitere Schritte einzuleiten. Ein Schritt kann zum Beispiel darin bestehen, den Thrombus nach misslungener oder unvollständige Aspiration mit einer selbstexpandierbaren Vorrichtung zur entfernen oder eine selbstexpandierbaren Vorrichtung, falls diese bereits benutzt wurde, ein weiteres Mal zum Thrombus zu führen (iteratives Rekanalisationsverfahren).

Die Erfindung ist nicht auf die Aspiration von Thromben oder auf die Thrombektomie beschränkt. Bei der Behandlung von Stenosen, im Herz, in der Peripherie aber besonders intrakraniell, durch Ballon oder durch einen Stent kann es vorteilhaft sein, dass kleine Plaquepartikel aspiriert werden, bzw. dass die Plaque-Dilatation unter Aspiration durchgeführt wird. Parallel ist es vorteilhaft, kalte Flüssigkeit zu verabreichen, um im Fall von distalen Gefäßverschlüssen durch abschwimmenden Plaquepartikel eine Neuroprotektion zu bewirken. Auch hier kann das Aspirationslumen im weitesten Sinn, also ohne Aspiration, eingesetzt werden, zB für die Zufuhr eines Stents. Weiterhin ist es möglich, Spasmen mit dem Katheter zu behandeln. Auch hier kann das Aspirationslumen eingesetzt werden, um einen Katheter durch den Spasmus zu schieben und eventuell eine Spasmusdilatation durch einen Ballon zu bewirken. Parallel kann durch das Infusionslumen kalte Flüssigkeit zur Kühlung verabreicht werden. Der Katheter kann auch bei der Behandlung von Aneurysmen und Malformationen benutzt werden. Auch hier wirkt sich die Kühlung im Fall von Blutungen neuroprotektiv aus, während das Aspirationslumen die Zufuhr von Kathetern für die Behandlung, z.B. durch Coils, Stents, Flow Diverters und/oder Kleber, ermöglicht.

Die Erfindung wird nachfolgend anhand der schematischen Zeichnungen mit weiteren Einzelheiten näher erläutert.

In diesen zeigen:
- Figur 1: einen zweilumigen Katheter in einem Blutgefäß vor einem Thrombus, bei dem gleichzeitig aspiriert und eine Infusion verabreicht wird;
- Figur 2: eine Längsschnitt durch den Katheter gemäß Figur 1;
- Figuren 3a, 3b, 3c: einen Querschnitt durch verschiedene Ausführungen eines zweilumigen Katheters, der für die Erfindung geeignet ist.
- Fig. 4: einen Längsschnitt durch den erfindungsgemäßen Katheter; und
- Fig. 5: einen Querschnitt durch einen Katheter nach einem weiteren erfindungsgemäßen Ausführungsbeispiel.

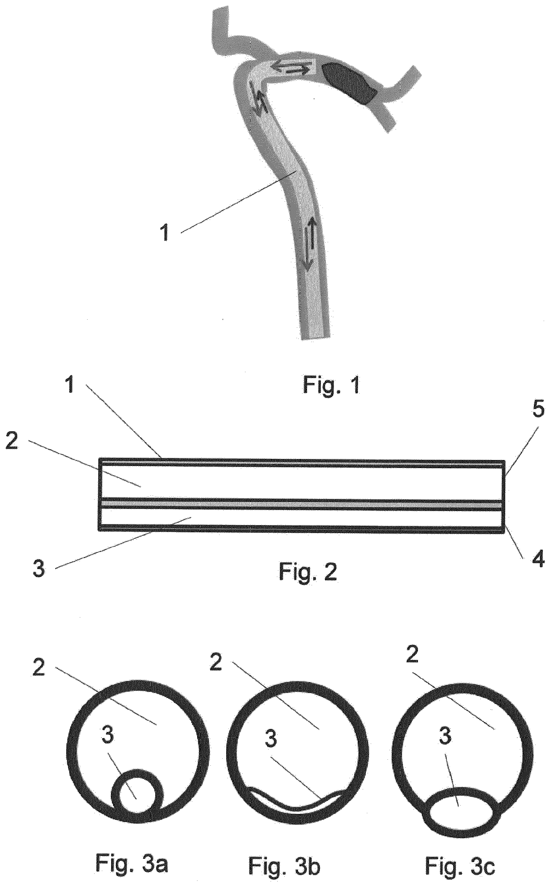

Figur 1 zeigt den Katheter 1 eines medizinischen Aspirationssystems nach einem erfindungsgemäßen Ausführungsbeispiel während der Behandlung. Dazu ist der Katheter 1 in ein Blutgefäß eingeführt. Die Katheterspitze befindet sich unmittelbar vor dem im Blutgefäß befindlichen Thrombus, der entfernt werden soll. Der Katheter 1 weist zwei Lumen auf. Das erste Lumen bildet ein Aspirationslumen 2, das mit einer Aspirationseinheit zum Absaugen des Thrombus verbunden ist (nicht dargestellt). Die Aspirationseinheit kann ein Sauggerät oder eine Spritze sein. Das zweite Lumen ist ein Infusionslumen 3, durch das ein Stoff, beispielsweise eine gekühlte Flüssigkeit dem Blutgefäß zugeführt wird. Das Infusionslumen 3 ist mit einer nicht dargestellten Funktionseinheit verbunden, die als Kühleinheit ausgebildet ist.

Wie in Figur 2 zu erkennen, weisen das Infusionslumen 3 eine Austrittsöffnung 4 und das Aspirationslumen eine Eintrittsöffnung 5 auf. Die Austrittsöffnung 4 und die Eintrittsöffnung 5 sind im Bereich der distalen Katheterspitze ausgebildet, die im Gebrauch im Blutgefäß zur Aspiration des Thrombus angeordnet ist. Konkret münden die Öffnungen 4, 5 in der Katheterspitze. Bei dem Ausführungsbeispiel gemäß Figur 2 sind die Austrittsöffnung 4 und die Eintrittsöffnung 5 auf derselben Höhe angeordnet. Dies bedeutet, dass die beiden Öffnungen 4, 5 im Wesentlichen in derselben Ebene angeordnet sind. Diese Anordnung bildet die distale Maximalposition, die die Austrittsöffnung 4 des Infusionslumens 3 bezogen auf die Eintrittsöffnung 5 des Aspirationslumens 1 einnehmen kann (Abstand = 0 cm). Abweichend von der in Figur 2 dargestellten Ausführung, ist die Austrittsöffnung 4 erfindungsgemäß in proximaler Richtung zurückversetzt angeordnet, so dass diese in proximaler Richtung von der Eintrittsöffnung 5 beabstandet ist. Je größer der Abstand zwischen der Eintrittsöffnung 5 und der Austrittsöffnung 4 ist, umso weniger gekühlte Flüssigkeit wird durch die Eintrittsöffnung 5 abgesaugt. Außerdem ist die Wahrscheinlichkeit größer, dass die kalte Infusion in Seitengefäße und Abzweigungen hineinfließt. Der Abstand zwischen den beiden Öffnungen 4, 5 sollte nicht mehr als 30 cm betragen, damit die Kühlwirkung im Bereich der Eintrittsöffnung 5 beibehalten wird.

Das Aspirationssystem erlaubt die gleichzeitige Aspiration des Thrombus und die Kühlung des Gefäßes. Dies ist durch die großen und kleinen Pfeile im Katheterangedeutet. Die großen, in proximaler Richtung weisenden Pfeile verdeutlichen den Absaugstrom, der durch den von der Aspirationseinheit erzeugten Unterdruck bewirkt wird. Der Absaugstrom führt dazu, dass der im Blutgefäß befindliche Thrombus in den Katheter, konkret in das Aspirationslumen 2 des Katheters 1 eingesaugt wird. Gleichzeitig erfolgt die Zufuhr eines Stoffes in das Blutgefäß. Der zuführende Stoffstrom wird durch die in distaler Richtung weisenden, kleineren Pfeile verdeutlicht. Der Stoffstrom wird durch eine nicht dargestellte Funktionseinheit erzeugt, die einen Überdruck generiert, durch den der Stoff in das Blutgefäß gefördert wird.

Die Aspiration und die Infusion müssen nicht zwingend gleichzeitig stattfinden. Die Infusion kann während der Aspiration unterbrochen werden. Selbst wenn die Infusion und die Aspiration nicht zeitgleich erfolgen, hat die vorliegende Ausführungsform den Vorteil gegenüber dem Stand der Technik, dass beide Phasen, d.h. die Phase der Aspiration und die Phase der Infusion mit ein und demselben Katheter im schnellen zeitlichen Wechsel ohne die Notwendigkeit eines Katheterwechsels durchgeführt werden können.

Vorzugsweise wird durch das Aspirationslumen nur aspiriert. Um zu vermeiden, dass durch das Aspirationslumen Infusionmittel zugeführt wird, kann das Aspirationslumen ausschließlich mit der Aspirationseinheit verbunden sein. Wenn das Aspirationssystem entsprechend vorsichtig gehandhabt wird, ist es auch möglich, das Aspirationslumen mit 2 Anschlüssen zu versehen, wobei ein erster Anschluss mit einer Aspirationseinheit und ein zweiter Anschluss mit einer Infusionseinheit verbunden sind. In dem Fall ist es zB möglich, Kontrastmittel durch das Aspirationslumen zu verabreichen, so dass in dem Infusionslumen weiterhin unbeeinflusst kalte Infusion fließt. Die Infusionseinheit ist in dem Fall zB eine Spritze. Wenn beispielsweise die Infusion nur vor der Aspiration stattfindet, besteht kein Risiko, dass im Aspirationslumen befindliche Thrombenteile ausgeschwemmt werden.

Bei der Kühleinheit handelt es sich um ein Kühlaggregat, in welchem die Kühlung der Flüssigkeit und die Erzeugung des Druckes für die Förderung der Flüssigkeit erfolgen. Anstelle des Kühlaggregats ist es auch möglich, einen einfachen gekühlten Beutel zu verwenden, der am mit der zu verabreichenden Flüssigkeit gefüllt ist.

Anstelle von Kühlmittel kann auch Lyse oder ein Kontrastmittel während der Aspiration verabreicht werden. Die Zufuhreinheit kann dabei eine einfache Spritze sein.

Figur 2 stellt einen Längsschnitt durch den Katheter gemäß Figur 1 dar, wobei der größere Durchmesser des Aspirationslumens 2 im Vergleich zum Infusionslumen 3 gut zu sehen ist.

Figuren 3a, 3b, 3c zeigen verschiedene Varianten des Katheters 1 gemäß Figur 1. Bei der Variante gemäß Figur 3a es sind das Aspirationslumen 2 und das Infusionslumen 3 exzentrisch angeordnet. Dies gilt auch für die Varianten gemäß Figuren 3b, 3c. Bei der Variante gemäß Figur 3a sind das Aspirationslumen 2 und das Infusionslumen 3 jeweils mit einem kreisförmigen Querschnitt ausgebildet. Bei der Variante 3b zeichnet sich das Infusionslumen 3 durch eine abschnittsweise flexible Wandung aus. Im Gebrauch, das heißt bei Beaufschlagen des Infusionslumens 3 mit einer Flüssigkeit bzw. mit Druck öffnet sich automatisch der Querschnitt des Infusionslumens 3. Alternativ kann die in Figur 3b gezeigte Wandung mit der dargestellten Geometrie stabil, d.h. nicht oder schwer verformbar sein.

Bei der Variante gemäß Figur 3c weist das Infusionslumen 3 einen ovalen Querschnitt auf. Das Infusionslumen 3 ist in die Wandung des Aspirationlumens 2 integriert. Es ist auch möglich, dass das Aspirationslumen 2 rund und das Infusionslumen 3 vollständig außerhalb des Aspirationslumens 2 angeordnet ist. Hierbei kann ein sichelförmiger Querschnitt möglich sein. Das Außenprofil kann rund sein oder einen Vorsprung aufweisen

In den Zeichnungen dargestellt ist das Merkmal, wonach die Zugangsöffnung des Infusionslumens 3 zumindest auf derselben Höhe wie die Zugangsöffnung der größeren Aspirationslumens 2 angeordnet ist. Die Zugangsöffnung des Infusionslumens 3 kann alternativ weiter proximal als die Zugangsöffnung des Aspirationslumens 2 angeordnet sein.

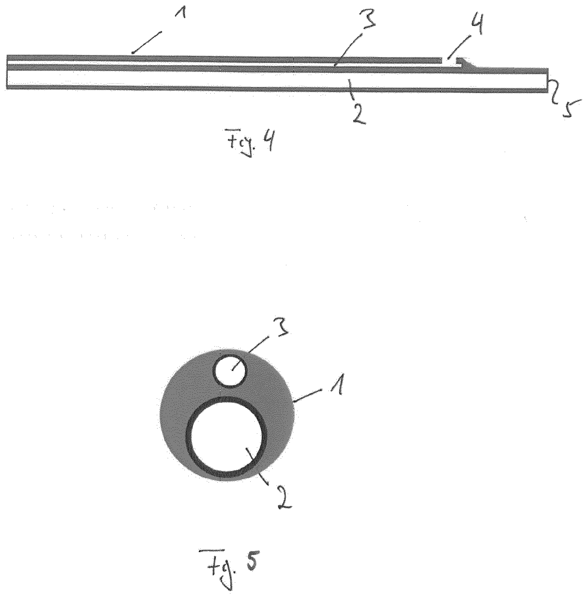

In diesem Fall kann das Infusionslumen 3 eine seitliche Öffnung bzw. Austrittsöffnung 4 aufweisen, wie in Figur 4 gezeigt. Diese kann ein einfaches Loch in der Katheteraußenwand darstellen. Das Infusionslumen 3 im distalen Bereich kann verschlossen werden, zB durch Kleben, ohne dass das Profil desAspirationslumens 2 sich dabei ändert. Alternativ kann das Aspirationslumen 2 distal zur Öffnung des Infusionslumens 3 größer sein, bzw eine größere Querschnittfläche aufweisen, weil die Kontur des Infusionslumens 3 (das nach Innen ragt) nicht mehr vorhanden ist oder ein kleineres Profil zeigt. Beispiele: Eine zweilumige Extrusion bzw. ein zweilumiger Schlauch oder zumindest eine ein Infusionslumen 3 aufweisende Extrusion oder ein entsprechender Schlauch erstreckt sich im proximalen Bereich bis zur Öffnung 4 des Infusionslumens 3. Ab der Öffnung 4 ist der proximale Bereich mit einer einlumigen Extrusion oder einem entsprechenden Schlauch, oder zumindest einer infusionslumenfreien Extrusion bzw. einem entsprechenden Schlauch verbunden. Es kann sein, dass der proximale Bereich in den distalen Bereich übergeht. Das ist z.B. dann möglich, wenn das Infusionslumen 3 mit einem Mandrel und entsprechendem gleichzeitiger Erwärmung nach Außen gedrückt wird. Es ist dann möglich, dass ein Teil des Profils des Infusionslumens 3 bestehen bleibt und nach Innen ragt. Es ist auch möglich, dass das Aspirationslumen 2 unverändert bleibt und durch die Reduzierung oder das Verschwinden des Infusionslumens 3 distal zur Öffnung der Außendurchmesser reduziert wird. Das ist insbesondere bei der Version mit einer separaten Verstärkung des Aspirationslumens 2 sinnvoll. In diesem Fall ragt das Infusionslumen 3 nicht nach Innen in das Aspirationslumen 2 hinein. Vielmehr befindet sich das Infusionslumen 3 außerhalb der Verstärkung. In diesem Fall kann das Lumen durch einen thermischen Prozess, zB durch Schrumpfen verschlossen werden, was den Außerdurchmesser des Katheters reduzieren würde. Entweder wird die Querschnittsfläche des Aspirationslumens 2 vergrößert, oder der Außendurchmesser des Katheters wird verkleinert distal zur Öffnung des Infusionslumens 2, oder beides. Alternativ bleiben beide unverändert.

Die Länge des Katheters kann mindestens 70 cm, insbesondere mindestens 80 cm, insbesondere mindestens 90 cm betragen (Position in der ACC - arteria carotis communis). Der Katheter kann alternativ eine Länge von mindestens 90 cm, insbesondere mindestens 100 cm, insbesondere mindestens 110 cm, insbesondere mindestens 120 cm, insbesondere mindestens 130 cm, insbesondere mindestens 140 cm aufweisen (Position in der ACI - arteria carotis interna, MCA - arteria cerebri media). Der Durchmesser des Katheters kann mindestens 7 Fr, insbesondere mindestens 8 Fr, höchstens 11 Fr, insbesondere höchstens 10 Fr, insbesondere höchstens 9 Fr betragen (Position in der ACC). Alternativ kann der Katheter einen Durchmesser zwischen 5 und 7 Fr, vorzugsweise von 6 Fr aufweisen (Position in der ACI, MCA).

Der Innendurchmesser des Aspirationslumens 2 beträgt ca. 300 µm weniger als der Außendurchmesser. Dabei ist der Außendurchmesser des gesamten Katheters gemeint. 300 µm ist beträgt die Wandung im Bereich der Untergrenze. Insbesondere beträgt der Unterschied zwischen 200 und 1200 µm, insbesondere zwischen 300 und 1000 µm, insbesondere zwischen 400 und 800 µm.

Zwar wird durch das Infusionslumen 3, das teilweise oder vollständig in das Aspirationslumen 2 hineinragt, der Strömungsquerschnitt des Aspirationslumens 2 entsprechend verringert. Bei den kleinen Dimensionen des Katheters 1, insbesondere des Infusionslumens 3 wird die Aspiration dadurch allerdings nicht wesentlich beeinträchtigt.

Alternativ ragt das Infusionslumen 2 nicht in das Aspirationslumen 3 hinein, wie in Fig. 5 gezeigt. Vielmehr sind beide Lumen 2, 3 seitlich angeordnet und in besten Fall rund. Sie sind umschlossen von einem runden Außenprofil. Um jedes Lumen 2, 3 ist im bevorzugten Fall eine Verstärkung angeordnet. Es kann sein, dass die Verstärkung nur um das Aspirationslumen 2 oder nur um das Infusionslumen 3 angeordnet ist. Eine Variante ohne Verstärkung ist auch möglich.

Das Infusionslumen 3 ist höchstens so groß, dass ein mindestens ein 3 FR Katheter, insbesondere mindestens ein 4 Fr Katheter durch das Aspirationslumen 2 transportierbar ist (für die Positionierung in der ACI/MCA). In der ACC sollte das verbleibende Aspirationslumen 2 für die Zufuhr eines 5 FR oder 6 FR Katheters ausgelegt sein. Für den hinteren Hirnkreislauf (arteria vertebralis und Basilararterie) sind dieselben Dimensionen wir für die Platzierung in der ACI denkbar.

Die Querschnittsfläche des Infusionslumens 3 kann zwischen 0,16 mm² und 0,4 mm² betragen. Insbesondere kann das Lumen einen Durchmesser zwischen 0,16 mm² und 1 mm² aufweisen, insbesondere zwischen 0,2 mm² und 0,8 mm², insbesondere zwischen 0,3 mm² und 0,6 mm².

Generell ist eine Kombination des Aspirationssystems mit einer Ballonkühlung möglich. Dies bedeutet, dass der Katheter mehr als zwei Lumen aufweist, beispielsweise vier oder fünf Lumen. Im Optimalfall weist der Katheter jedochgenau zwei Lumen auf. Dadurch können die Dimensionen des Katheters miniaturisiert werden, so dass eine gute Aspiration in kleinen intrakraniellen Gefäßen möglich ist.

Die Kombination des Aspirationssystems mit einem Verschlussballon ist ebenfalls möglich. Der Verschlussballon weist eine gute Compliance mit der Gefäßwand auf.

Der Okklusionsballon kann proximal zu beide Öffnungen 4, 5 positioniert werden, wenn beide Öffnungen 4, 5 auf der gleichen Höhe an der Spitze des Katheters angeordnet sind. Wenn die Öffnung 4 des Infusionslumens proximal angeordnet ist, kann sich der Okklusionsballon dazwischen befinden. Dadurch ist es möglich, dass die kalte Infusion während der Aspiration nicht mitaspiriert wird und in proximal gelegene Kollateralgefäße strömt. Alternativ kann die proximal gelegene Öffnung 4 auch distal zum Okklusionsballon angeordnet sein, wenn es erforderlich ist, dass die Kühlung auch während der Okklusion distal fließt.

Das Infusionslumen 3 kann abweichend von Fig. 2 weiter proximal enden. Das Aspirationslumen 2 wird dadurch am distalen Ende flexibler. Eine distale Verkleinerung des Außendurchmessers, besonders im Fall der proximalen Öffnung des Infusionslumens 3, ist möglich. Das Aspirationslumen 2 kann dabei die gleiche Dimension beibehalten. Auch in anderen Ausführungen mit Öffnung auf gleicher Höhe ist eine Reduzierung des Außendurchmessers möglich.

Das Aspirationssystem erlaubt den Einsatz von hypertonen Lösungen, auch als kalte Flüssigkeit, zur Ödem-Reduzierung. Durch das Infusionslumen 3 können Medikamente aller Art verabreicht werden. Die Aspirationseinheit kann ein System mit Druckeinheit, d.h. eine Pumpe oder eine Spritze aufweisen.

Das Aspirationslumen 2 kann dazu genutzt werden, um eine Rekanalisationseinrichtung und/oder Thrombektomie-Einheit zuzuführen. Im Zusammenhang mit dem erfindungsgemäßen Aspirationssystem und allen Ausführungsbeispielen dieser Anmeldung wird daher als weiteres Ausführungsbeispiel die Kombination des Katheters 1 mit einer Rekanalisationseinrichtung und/oder Thrombektomie-Einheit offenbart und beansprucht.

Alternativ kann das Aspirationslumen 2 dazu genutzt werden, um einen Mikrokatheter mit einem selbstexpandierbaren Thrombektomie-Element oder einem selbstexpandierbaren Rekanalisation-Element zuzuführen. Im Zusammenhang mit dem erfindungsgemäßen Aspirationssystem und allen Ausführungsbeispielen dieser Anmeldung wird daher als weiteres Ausführungsbeispiel die Kombination des Katheters 1 mit einem weiteren Aspirationskatheter bspw. in der Form eines Sets mit oder ohne weitere selbstexpandierende Rekanalisationsvorrichtung offenbart. Der weitere Aspirationskatheter kann eine Größe von 5 Fr oder 6 Fr haben oder ähnliches. In diesem kann die Rekanalisationsvorrichtung und/oder das Thrombektomie-Element angeordnet sein.

## Patentansprüche

1. Medizinisches Aspirationssystem mit einem Katheter (1) mit wenigstens zwei Lumen, wobei ein erstes Lumen ein Aspirationslumen (2) bildet, das mit einer Aspirationseinheit zum Absaugen von Stoffen aus einem Blutgefäß durch eine Eintrittsöffnung (5) des Aspirationslumens (2) verbunden ist, und ein zweites Lumen ein Infusionslumen (3) bildet, durch das ein Stoff dem Blutgefäß durch eine Austrittsöffnung (4) des Infusionslumens (3) zuführbar ist, wobei das Infusionslumen (3) mit einer Kühleinheit verbunden ist, die eine gekühlte Flüssigkeit zur Verabreichung durch das Infusionslumen (3) bereitstellt,
**dadurch gekennzeichnet, dass**
die Austrittsöffnung (4) des Infusionslumens (3) in einem Bereich zwischen der Eintrittsöffnung (5) des Aspirationslumens (2) und einer Position proximal zur Eintrittsöffnung (5) des Aspirationslumens (2) angeordnet ist.

2. System nach Anspruch 1,
**dadurch gekennzeichnet, dass**
das Aspirationslumen (2) und das Infusionslumen (3) durch eine ventilfreie Wandung fluiddicht getrennt sind.

3. System nach Anspruch 1 oder 2,
**dadurch gekennzeichnet, dass**
das Aspirationslumen (2) und das Infusionslumen (3) einteilig ausgebildet sind.

4. System nach einem der Ansprüche 1 bis 3,
**dadurch gekennzeichnet, dass**
der Bereich zwischen der Eintrittsöffnung (5) des Aspirationslumens (2) und der Position proximal zur Eintrittsöffnung (5) des Aspirationslumens (2) wenigstens 5 cm beträgt.

5. System nach einem der vorhergehenden Ansprüche,
**dadurch gekennzeichnet, dass**
der Bereich zwischen der Eintrittsöffnung des Aspirationslumens (2) und der Position proximal zur Eintrittsöffnung des Aspirationslumens (2) höchstens 30 cm beträgt.

6. System nach einem der vorhergehenden Ansprüche,
**dadurch gekennzeichnet, dass**
die Länge des Katheters (1) mindestens 80 cm beträgt.

7. System nach einem der vorhergehenden Ansprüche,
**dadurch gekennzeichnet, dass**
der Durchmesser des Katheters (1) mindestens 7 Fr beträgt.

8. System nach einem der vorhergehenden Ansprüche,
**dadurch gekennzeichnet, dass**
der Durchmesser des Katheters (1) höchstens 11 Fr beträgt.

9. System nach einem der vorhergehenden Ansprüche
**dadurch gekennzeichnet, dass**
die Querschnittsfläche des Infusionslumens (3) zwischen 0,16 mm² und 0,4 mm² beträgt.

10. System nach einem der Ansprüche 1 bis 9,
**dadurch gekennzeichnet, dass**
die wenigstens zwei Lumen des Katheters (1) ineinander längsverschieblich angeordnet sind.

11. System nach Anspruch 10,
**dadurch gekennzeichnet, dass**
die wenigstens zwei Lumen des Katheters (1) koaxial ineinander längsverschieblich angeordnet sind.

12. System nach Anspruch 10 oder 11,
**dadurch gekennzeichnet, dass**
das Aspirationslumen oder das Instrumentenlumen koaxial innerhalb des Infusionslumens angeordnet ist.

## Claims

1. A medical aspiration system having a catheter (1) with at least two lumens, wherein a first lumen forms an aspiration lumen (2) which is connected to an aspiration unit for removing substances from a blood vessel by suction through an inlet opening (5) of the aspiration lumen (2), and a second lumen forms an infusion lumen (3) through which a substance can be supplied to the blood vessel through an outlet opening (4) of the infusion lumen (3), wherein the infusion lumen (3) is connected to a cooling unit which provides a cooled liquid for administration through the infusion lumen (3),
**characterized in that** the outlet opening (4) of the infusion lumen (3) is disposed in a region between the inlet opening (5) of the aspiration lumen (2) and a position proximal to the inlet opening (5) of the aspiration lumen (2).

2. The system as claimed in claim 1, **characterized in that** the aspiration lumen (2) and the infusion lumen (3) are separated in a fluid-tight manner by means of a wall without valves.

3. The system as claimed in claim 1 or claim 2, **characterized in that** the aspiration lumen (2) and the infusion lumen (3) are formed in one piece.

4. The system as claimed in one of claims 1 to 3, **characterized in that** the region between the inlet opening (5) of the aspiration lumen (2) and the position proximal to the inlet opening (5) of the aspiration lumen (2) is at least 5 cm in length.

5. The system as claimed in one of the preceding claims, **characterized in that** the region between the inlet opening of the aspiration lumen (2) and the position proximal to the inlet opening of the aspiration lumen (2) is at most 30 cm in length.

6. The system as claimed in one of the preceding claims, **characterized in that** the length of the catheter (1) is at least 80 cm.

7. The system as claimed in one of the preceding claims, **characterized in that** the diameter of the catheter (1) is at least 7 Fr.

8. The system as claimed in one of the preceding claims, **characterized in that** the diameter of the catheter (1) is at most 11 Fr.

9. The system as claimed in one of the preceding claims, **characterized in that** the cross-sectional area of the infusion lumen (3) is between 0.16 mm² and 0.4 mm².

10. The system as claimed in one of claims 1 to 9, **characterized in that** the at least two lumens of the catheter (1) are disposed longitudinally displaceably one inside the other.

11. The system as claimed in claim 10, **characterized in that** the at least two lumens of the catheter (1) are disposed longitudinally displaceably and coaxially one inside the other.

12. The system as claimed in claim 10 or claim 11, **characterized in that** the aspiration lumen or the instrument lumen is disposed coaxially inside the infusion lumen.

## Revendications

1. Système d'aspiration médical comportant un cathéter (1) doté d'au moins deux lumens, un premier lumen constituant un lumen d'aspiration (2) qui est connecté à une unité d'aspiration pour aspirer des substances dans un vaisseau sanguin à travers un orifice d'entrée (5) du lumen d'aspiration (2), et un second lumen constituant un lumen de perfusion (3) à travers lequel une substance peut être acheminée dans le vaisseau sanguin par un orifice de sortie (4) du lumen de perfusion (3), le lumen de perfusion (3) étant raccordé à une unité de refroidissement qui met à disposition un liquide refroidi pour administration par le lumen de perfusion (3),
**caractérisé en ce que** l'orifice de sortie (4) du lumen de perfusion (3) est disposé dans une zone située entre l'orifice d'entrée (5) du lumen d'aspiration (2) et une position proximale par rapport à l'orifice d'entrée (5) du lumen d'aspiration (2).

2. Système selon la revendication 1, **caractérisé en ce que** le lumen d'aspiration (2) et le lumen de perfusion (3) sont séparés de manière étanche au fluide par une paroi sans vanne.

3. Système selon la revendication 1 ou 2, **caractérisé en ce que** le lumen d'aspiration (2) et le lumen de perfusion (3) forment une seule pièce.

4. Système selon une des revendications 1 bis 3, **caractérisé en ce que** la zone située entre l'orifice d'entrée (5) du lumen d'aspiration (2) et la position proximale par rapport à l'orifice d'entrée (5) du lumen d'aspiration (2) mesure au moins 5 cm.

5. Système selon une des revendications précédentes, **caractérisé en ce que** la zone située entre l'orifice d'entrée du lumen d'aspiration (2) et la position proximale par rapport à l'orifice d'entrée du lumen d'aspiration (2) mesure au plus 30 cm.

6. Système selon une des revendications précédentes, **caractérisé en ce que** la longueur du cathéter (1) est d'au moins 80 cm.

7. Système selon une des revendications précédentes, **caractérisé en ce que** le diamètre du cathéter (1) est d'au moins 7 Fr.

8. Système selon une des revendications précédentes, **caractérisé en ce que** le diamètre du cathéter (1) est d'au plus 11 Fr.

9. Système selon une des revendications précédentes **caractérisé en ce que** la surface de section transversale du lumen de perfusion (3) est comprise entre 0,16 mm² et 0,4 mm².

10. Système selon une des revendications 1 bis 9, **caractérisé en ce que** les au moins deux lumens du cathéter (1) sont disposés de manière à être déplaçables longitudinalement l'un dans l'autre.

11. Système selon la revendication 10, **caractérisé en ce que** les au moins deux lumens du cathéter (1) sont disposés de manière à être déplaçables longitudinalement l'un dans l'autre coaxialement.

12. Système selon la revendication 10 ou 11, **caractérisé en ce que** le lumen d'aspiration ou le lumen instrumental est disposé coaxialement dans le lumen de perfusion.
